# EUROPEAN PATENT APPLICATION

(11) **EP 4 756 472 A1**
(43) Date of publication of application: **10.06.2026**
(21) Application number: 24218340.8
(22) Date of filing: 09.12.2024
(51) Int. Cl.: G01R 33/28, G01R 33/422

(54) **IN-BORE COOLING FOR MAGNETIC RESONANCE APPARATUS**

(71) Applicant: Koninklijke Philips N.V., 5656 AE Eindhoven (NL)
(72) Inventor: VERNICKEL, Peter, Eindhoven (NL); LIPS, Oliver, Eindhoven (NL); LEUSSLER, Christoph Günther, Eindhoven (NL)
(74) Representative: Philips Intellectual Property & Standards

(57) **Abstract**

The present invention provides a cooling system (1) for an in-bore equipment of a medical apparatus, comprising: a compact fan (11) which is resistant to magnetic interference, a radio frequency, RF, shield (12) comprising a RF shielding housing (111) which completely encloses the compact fan (11), wherein the housing (111) comprises an air-in duct (112) and an air-out duct (113), and RF shielding mesh parts (121, 122) covering the air-in duct (112) and the air-out duct (113). In this way, a solution for efficient and localized cooling inside the bore of the medical apparatus is provided, to enhance the performance, the efficiency of medical apparatus and to improve the patient comfort.

## Description

### FIELD OF THE INVENTION

The invention relates to the field of magnetic resonance (MR) apparatus, and in particular to the cooling and ventilation of MR apparatus.

### BACKGROUND OF THE INVENTION

Medical imaging devices, such as Magnetic Resonance Imaging (MRI) and Computed Tomography (CT) systems, are at the forefront of diagnostic medicine, providing critical insights into internal structures and functions. Typically, the medical imaging device includes a bore which is a core, cylindrical part of the machine where the patient is positioned during the scanning or imaging procedure. The bore houses critical imaging components and plays a central role in generating diagnostic images. These critical imaging components, including magnets, X-ray tubes, gradient coils, and radiofrequency systems, produce considerable heat as they operate, making effective cooling and ventilation essential for performance, safety, and patient comfort. Moreover, the compact size of the bore in medical imaging devices poses significant challenges for effective ventilation and airflow management.

Conventional cooling systems in these devices typically include air-cooled and liquid-cooled mechanisms. Air-cooled systems use fans and ventilation pathways to dissipate heat generated by components such as electronics. This method is straightforward, cost-effective, and commonly used where the space for airducts is available or magnetic stray fields of the fans do not interfere with the functioning of the imaging system and vice versa. Liquid-cooled systems, on the other hand, are more advanced and involve circulating coolants like water or specialized fluids through heat exchangers to absorb and remove heat. These systems are essential for devices with strong dissipation or high demands on temperature stability, particularly those gradient coils or amplifiers that require precise temperature control for optimal performance.

In practice, the strong static magnetic field in the MR apparatus precludes the use of conventional electric motor-driven fans, as the magnetic fields of the motor and the scanner would interfere with one another. While electrostatic motors are an alternative, they are prone to operational errors and typically lack sufficient power. Consequently, conventional air-cooled systems for the bore of the medical imaging system typically engage a remote fan coupled with an air duct system, which delivers a uniform airflow throughout the bore longitudinally, minimizing heat and humidity buildup generated by the equipment or the patient. Additionally, they may rely on external air conditioning units to cool and dehumidify the air before it is circulated through the bore.

However, this setup poses challenges for efficient cooling of electronic components within the bore. Moreover, the airflow often fails to adequately reach the patient, who is typically surrounded by coil arrays that obstruct the airflow. These limitations hinder the overall effectiveness of cooling and ventilation, impacting both device performance and patient comfort.

### SUMMARY OF THE INVENTION

It is an object of the invention to provide solutions for efficient and localized cooling inside the bore of a medical apparatus to enhance the performance and efficiency of the medical apparatus and to improve the patient comfort.

According to the invention, this object is addressed by the subject matter of the independent claims. Embodiments of the invention are described in the dependent claims.

Therefore, according to the first aspect of the invention, a cooling system is provided. The cooling system is suitable for cooling and/or ventilation for a medical apparatus. The cooling system comprises a compact fan which is resistant to magnetic interference, a radio frequency (RF) shield including an RF shielding housing enclosing the compact fan completely. Specifically, the housing comprises an air-in duct and an air-out duct, and RF shielding mesh parts covering the air-in duct and the air out duct. The medical apparatus may be one of an MR apparatus, CT apparatus, a single photon emission computed tomography (SPECT) apparatus and a positron emission tomography (PET) apparatus.

By providing a compact fan which is resilient or immune to the magnetic interference resulted from the MR apparatus, the cooling system enables stable, efficient and localized cooling and/or ventilation for the equipment and the patient during the medical imaging process. In addition to the magnetic interference resistance of the compact fan, the RF shield provides additional protection against magnetic interference at radio frequency that could be induced by components of MR apparatus, thus enhancing the magnetic interference resistance of the compact fan and ensuring the robustness of the compact fan during operation. This applies also vice versa, it is ensured that the MR apparatus and its operation is not affected by magnetic interference resulting from a cooling system.

Specifically, apart from the RF shielding mesh covering the air-in and air-out ducts provided in the housing, the RF shield housing comprise RF shielding paint or foil covering the rest of the housing. This ensures proper air circulation and dustproofing for the compact fan while providing additional protection to radio frequency electromagnetic interference.

Here, the RF shield is provided to avoid RF signal from leaking out and in and may be designed to be impenetrable by RF signal near Larmor frequency. At the same time, it may remain transparent to electromagnetic (EM) signals within the gradient frequency range. This balance can be achieved using established RF design techniques, which include optimizing the shield geometry and material properties.

Optionally, the size of each mesh part may be kept below 4 cm × 4 cm. The limitation of the RF shield's screen part size facilitates the effective blocking of the RF signals while allowing the passage of lower-frequency gradient-related signals. This design approach helps maintain the integrity of the MRI system's imaging capabilities and ensures compliance with operational requirements.

The cooling system may be applied directly inside a bore of the MR apparatus, specifically, the cooling system may be applied or arranged directly in an in-bore equipment used with the MR apparatus. The cooling system is also suitable to be used in a CT gantry.

Here, the bore refers to the core, approximately cylindrical part of the medical imaging device where the patient is positioned during the scanning or imaging procedure. The term "in-bore equipment" refers to equipment used inside the bore during the scanning or imaging procedure. For example, the in-bore equipment may include at least one of the following: coil arrays, patient tables, mirror systems, positioning aides, equipment for patient support and similar components.

The arrangement of the compact fan may be varied according to the configuration of the in-bore equipment and the needs of the patient, making it possible to control the direction of the airflow based on practical requirements.

The cooling system could also be used in combination with conventional ventilation systems that engage remote fans and a uniform airflow to further enhance the cooling and ventilation efficiency.

According to one embodiment of the invention, the compact fan may be a solid-state fan. The solid-state fan is a cooling device that operates without the known rotating parts of electric motors like magnets, ferrites, and inductor coils, utilizing technologies like electrostatic or thermoelectric systems to generate airflow. This design makes the solid-state fan resistant to magnetic interference, enhances reliability, reduces noise, and minimizes maintenance requirements, making it ideal for applications where traditional fans may fail due to mechanical wear or electromagnetic interference. Moreover, as no rotating parts are employed, the cooling system with a solid-state fan is also suitable for cooling in a CT gantry.

According to one embodiment of the invention, the cooling system may further comprise a temperature measuring unit, a control unit. These components may be arranged inside the housing of the compact fan as well. With this configuration, the power supply and the control of the compact fan are executed locally, which further avoids the leaking of signals via the supply lines which may interfere with the MR apparatus.

According to one embodiment of the invention, the cooling system may further comprise a temperature measuring unit, a control unit, a power supply unit, and/or an RF filter unit connecting to the scanner system for interaction of scanner and fan system. The temperature measuring unit may be configured to measure the local temperatures of a component to be cooled, the air-in duct, the air-out duct, and/or the ambient temperature of the MR apparatus. The control unit may be configured to control the operation of the compact fan based on the measured temperature or other equipment parameters, such as a specific absorption rate (SAR) for an MR scanner (the SAR rate comes from the MR apparatus). The control unit may also be configured to communicate with the MR apparatus and/or control a detuning of the MR apparatus. The control unit could control the compact fan by adjusting the power provided for operating the compact fan. The power supply unit may be configured to supply power to the compact fan and the other components. The RF filter unit may be configured to further filter the RF signals to avoid radio frequency magnetic interference.

According to a second aspect of the invention, an in-bore equipment with a cooling system as discussed above is provided. As discussed above, by providing the cooling system to the in-bore equipment directly, stable, efficient and localized cooling and ventilation are provided.

Optionally, the compact fan may be attached to one surface of the in-bore equipment and/or may be arranged inside the in-bore equipment.

Optionally, the compact fan may be attached to electronics of the in-bore equipment to enable heat dissipation of electronics while providing cooling and ventilation effect.

Optionally, the electronics may comprise a printed circuit board (PCB), the compact fan may be attached to one side of the PCB inside the in-bore equipment. The air flows through the air-in and air-out ducts, passing over the components packaged on the other side of the PCB to achieve heat dissipation. This arrangement facilitates the compact design of the in-bore equipment and provides the possibility for simultaneous heat dissipation of the electronics and ventilation for patient comfort.

Additionally, air channels may be provided in combination with the cooling system of the in-bore equipment to guide and control the air flows.

According to an embodiment of the invention, the compact fan may be arranged in an in-bore equipment with its air-in duct adjacent to the outer periphery of the in-bore equipment, and the cooling system may comprise an air channel connecting the air-out duct of the compact fan and a surface of the in-bore equipment to guide an air flow from the compact fan to the surface. The surface may be a surface which is in contact with the body of a patient and is adapted to form an equipment-to-patient interface. The air channel connects the air-out duct and an opening on the surface.

Multiple air channels may be provided to connect the air-out duct and different openings on the surface to further enhance the cooling and ventilation effect.

Specifically, the air-in duct of the compact fan may be arranged parallel to one side of the outer periphery and in combination with the exterior to guide fresh air toward the equipment-to-patient interface for cooling and/or ventilation.

In a specific example, the air-in duct may be positioned approximately perpendicular to the longitudinal direction of the bore and faces away from the exit of the bore. In other words, the air-in duct of the compact fan may be arranged to face a main airflow direction inside the bore. This arrangement allows the main airflow to enter the air-in duct of the compact fan and be distributed toward the equipment-to-patient interface. In this context, the main airflow direction refers to the flow direction of the uniform airflow inside the bore, which is parallel to the longitudinal direction of the bore and directed toward the exit of the bore. The uniform airflow may be produced by a remote fan of conventional cooling technique.

According to another embodiment, the compact fan may be arranged, or the cooling system may comprise another compact fan which is arranged in the in-bore equipment with its air-out duct adjacent to the outer periphery of the in-bore equipment. The cooling system may comprise an air channel communicating the air-in duct of this compact fan and the surface of the in-bore equipment to guide an air flow from the surface to the compact fan. This surface may also be the surface in contact with the body of the patient and the air channel communicates the air-in duct and an opening on the surface.

Similarly, multiple air channels connecting different openings on the surface and the air-in ducts may be provided to further enhance the cooling and ventilation effect.

Specifically in this embodiment, the air-out duct of the compact fan may be arranged adjacent and/or parallel to one side of the outer periphery and in combination with the exterior to guide airflow exiting the bore for cooling and/or ventilation.

In a specific example, the air-out duct may be positioned approximately perpendicular to the longitudinal direction of the bore and towards the exit of the bore. In other words, the air-out duct of the compact fan may be arranged adjacent and/or parallel to a side of the outer periphery, which faces away from the main airflow direction. This arrangement allows for heat dissipation and humidity adjustment between the in-bore equipment and patient and facilitates the expulsion of heated air.

In these embodiments, the compact fan may be arranged adjacent to the outer periphery while being attached to the electronics of the in-bore equipment if the electronics are also arranged close to the outer periphery of the in-bore equipment.

According to an embodiment of the invention, the compact fan may be arranged inside the in-bore equipment, the cooling system may comprise an air channel connecting the air-in duct to one surface of the in-bore equipment and another air channel connecting the air-out duct to an opposite surface of the in-bore equipment. Corresponding openings may be provided on both surfaces.

In one specific example, these two surfaces may be horizontal surfaces which are opposite to each other. The air-in duct may be arranged facing the horizontal surface towards the patient to exhaust the warm air from the gap between the patient and the in-bore equipment, the air-in duct can also be arranged facing the horizontal surface away from the patient body to bring in the fresh air to the patient.

In another specific example, these two surfaces are opposite surfaces perpendicular to the longitudinal direction of the bore. The air-in duct may be connected to the perpendicular surface away from the exit of the bore, while the air-out duct may be connected to the opposite surface close to the exit of the bore. In other words, the surface in combination with the air-in duct may be the surface facing the main airflow direction, and the opposite surface in combination with the air-out duct may be the surface facing away from the main airflow direction.

Alternatively, these two surfaces are side surfaces that are parallel to the longitudinal direction of the bore and opposite to each other, these arrangements enable the radial air circulation inside the bore.

Similarly, in these embodiments, the compact fan may be attached to the electronics of the in-bore equipment. In this case, exterior air is guided to pass over the electronics, via the compact fan, from one side to the other side for efficient heat dissipation and simultaneous cooling and/or ventilation.

According to an embodiment of the invention, when the in-bore equipment comprises electronics, the compact fan may be arranged inside the in-bore equipment, the cooling system may comprise an air channel circulating the electronics of the in-bore equipment. The air channel may connect the air-in duct and the air-out duct to guide the airflow circulating the electronics. For a more compact design, the compact fan may be attached to the electronics placed inside the in-bore equipment. Specifically, the compact fan may be attached to the backside of the PCB of the electronics, or directly to the most dissipating components like a heat sink-. This is because the electronics may have hot spots where the temperature is higher than in most of the remaining areas. By creating an internal circulation flow, heat can be distributed evenly across the electronics and other components, allowing for greater overall heat dissipation to the surroundings. With proper air-guiding channels, it is also possible to improve the local dissipation of heat from the electronics. The internal air circulation is also beneficial for in-bore equipment that needs to be kept sealed, such as for sterilization.

According to another embodiment of the invention, when the in-bore equipment comprises electronics, the compact fan may be arranged in the in-bore equipment with its air-in duct adjacent to the outer periphery of the in-bore equipment. The cooling system may further comprise an air channel connecting the air-out duct, the electronics and an opposite side of the in-bore equipment to guide an air flow passing the electronics.

Similarly, the air-in duct may be arranged to face a main airflow direction inside the bore and the air-out duct may be connected to a side facing the exit of the bore so as to cool the electronics with air from the main air flow.

According to an embodiment of the present invention, multiple compact fans may be provided at different locations of the in-bore equipment. For example, the cooling system may comprise compact fans arranged with its air-in duct and/or air-out duct adjacent to the outer periphery of the in-bore equipment which respectively guide the airflow to the equipment-to-patient surface or exit the bore. The cooling system may also comprise compact fans arranged close to the outer periphery and inside the in-bore equipment respectively to improve the cooling of the electronics. These arrangements further improve the cooling and/or ventilation efficiency or may be employed to improve compatibility with the imaging systems. In a CT apparatus, the fan may be moved out of the field of view.

As introduced above, the in-bore equipment may refer to an equipment that could be used within the bore of the MR apparatus. Specifically, the in-bore equipment may include at least one of the following: coil arrays, a patient table, an equipment for patient support, and similar equipment.

According to a third aspect of the invention, an MR apparatus comprising an in-bore equipment as disclosed above is provided. The in-bore equipment may be integrated to the MR apparatus, or simply could be used in combination with MR apparatus during scanning. For example, the in-bore equipment may include at least one of the following: coil arrays, a patient table, an equipment for patient support, or similar equipment.

According to a fourth aspect of the invention, a method for operating the magnetic resonance apparatus as described above is provided. The method proposes operating the cooling system of the MR apparatus based on the monitored temperature and comprises the steps of monitoring local and/or ambient temperature of the MR apparatus, switching on and off the compact fan and/or adjusting the power provided for operating of the compact fan based on the monitored temperature.

The local temperature may be the temperature of a component to be cooled, the temperature measured at the air-in and/or air-out duct of compact fan and the similar. The component may be certain parts of the in-bore equipment, for example, the preamplifiers, detuning and digitization circuits, RF traps, power converters and or microcontrollers integrated to the in-bore equipment. The ambient temperature may be measured in the bore or in the gap between the patient and the in-bore equipment.

According to a specific embodiment of the invention, the cooling system may be controlled based on a specific absorption rate (SAR) of a scan session. In this embodiment, the method may monitor the SAR of the scan, switch the compact fan on or off based on the monitored SAR of the scan, and/or adjust the power provided for operating the compact fan based on the monitored SAR of the scan.

The SAR refers to the amount of radiofrequency energy absorbed by body tissues during an MRI scan. It is an important safety parameter to ensure that patients are not exposed to excessive RF energy, which can cause tissue heating. For scans with higher SAR levels, the power of the compact fan may be increased to enhance cooling and ventilation.

Alternately, the cooling system of the MR apparatus may be controlled based on an MR sequence applied for the scan. In this embodiment, the method may determine the applied MR sequence of the scan, switch the compact fan on or off based on the applied MR sequence, and/or adjust the power provided for operating the compact fan based on the applied MR sequence.

The MR sequence refers to a set of parameters and instructions used to acquire MRI data. It may define how the scanner manipulates magnetic fields and radiofrequency pulses to create images. Different sequences may be tailored for various anatomical regions, contrast types, and clinical purposes. With this approach, it is possible to control the compact fan differently for various scanning conditions, thus avoiding potential interference during scanning and ensuring image quality.

According to another embodiment of the present invention, the cooling system of the MR apparatus may also be controlled according to a remote temperature model. The method proposes monitoring a real-time safety margin with respect to the remote temperature model and adjusting the power provided for operating the compact fan by the control unit based on the real-time safety margin.

The remote temperature model may refer to a system or method used to estimate or monitor the temperature of the MR apparatus without direct contact with the area being measured. For example, these models may determine the temperature based on indirect signals such as infrared radiation, electrical resistance changes, or other physical properties influenced by temperature. The real-time safety margin may refer to the difference between the real-time temperature and the maximum allowable temperature. It ensures safe operation and avoids risks such as overheating, equipment damage, or harm to individuals.

According to this embodiment, the method may further propose sending interrupt information to the MR apparatus, in response to detecting that the real-time safety margin is smaller than a threshold. For example, the threshold may be set to ensure the temperature stays at least 1-5°C below the maximum allowable temperature. Once the real-time safety margin becomes smaller than the set threshold, the scanning is interrupted for safety. Additionally, the interruption may be executed by raising a detuning error in the case of an MR scanner.

The cooling system of the MR apparatus may be controlled by a central processor of the MR system or a control unit of the cooling system.

### BRIEF DESCRIPTION OF THE DRAWINGS

These and other aspects of the invention will be apparent from and elucidated with reference to the embodiments described hereinafter. Such an embodiment does not necessarily represent the full scope of the invention. In the drawings:
Fig. 1 schematically depicts a side cross-sectional view of a conventional MR apparatus with a bore;
Fig. 2 schematically depicts a block diagram of the cooling system according to an embodiment of the present invention;
Fig. 3 schematically depicts a block diagram of the cooling system according to another embodiment of the present invention;
Fig. 4 schematically depicts a side cross-sectional view of an in-bore equipment showing arrangements of the cooling system according to an embodiment of the present invention;
Fig. 5 schematically depicts a side cross-sectional view of the in-bore equipment showing arrangements of the cooling system according to another embodiment of the present invention;
Fig. 6 schematically depicts a side cross-sectional view of the in-bore equipment showing arrangements of the cooling system according to another embodiment of the present invention;
Fig. 7 schematically depicts a side cross-sectional view of the in-bore equipment showing arrangements of the cooling system according to another embodiment of the present invention;
Fig. 8 schematically depicts a flow chart of the methods operating the cooling system according to an embodiment of the present invention;
Fig. 9 schematically depicts a flow chart of the method operating the cooling system according to another embodiment of the present invention.

### DETAILED DESCRIPTION OF EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alteration and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

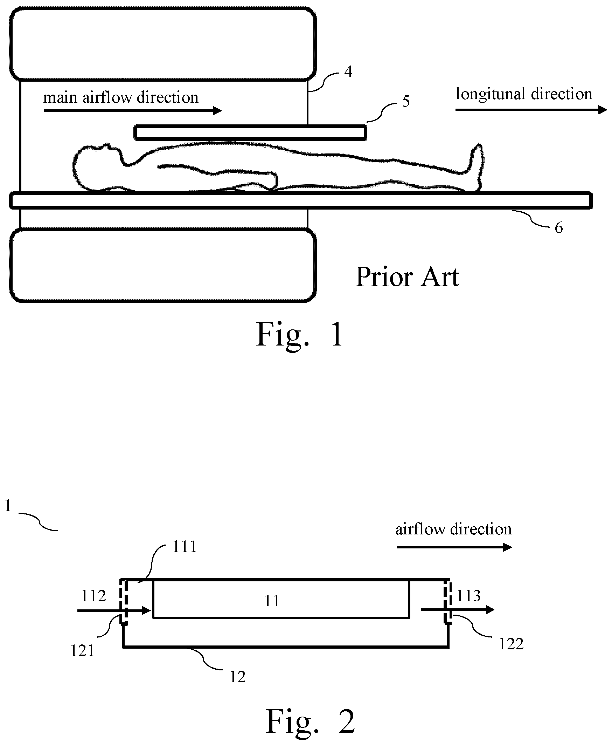

Fig. 1 depicts an MR apparatus with a conventional air-cooled system for the bore 4. The conventional air-cooled system employes a remote fan coupled with an air duct system, which delivers a uniform airflow at a single velocity throughout the bore 4 longitudinally. The main airflow direction is in parallel with the longitudinal direction of the bore 4.

It can be seen that the airflow may fail to adequately reach the patient, who is typically surrounded by coil arrays 5, thus the cooling and ventilation is not efficient.

Fig. 2 depicts a cooling system according to an embodiment of the present invention. The cooling system comprises a compact fan 11 which is resistant to magnetic interference; an RF shield 12 comprising an RF shielding housing 111. The housing 111 comprises an air-in duct 112 and an air-out duct 113, and RF shielding mesh parts 121, 122 covering the air-in duct 112 and the air-out duct 113.

The RF shield 12 may further comprises RF shielding paint or foil covering the rest of the housing 111, in addition to the RF shielding mesh parts 121, 122 covering the air-in and air-out ducts provided in the housing 111, thus ensuring proper air circulation for the compact fan while providing protection against leakage of signals in and out of the housing 111.

The compact fan 11, which is resistant to magnetic interference, can be a solid-state fan. In this example, a commercially available solid-state fan, such as Frore Systems' AirJet, can be utilized. The device is approximately the size of a credit card and employs solid-state technology, generating airflow through a unique combination of cavity and membrane design, along with electronic drive signals.

The fact that the solid-state fan does not use ferromagnetic parts or generate eddy currents makes it suitable for application inside the bore 4. Moreover, the compact size of the solid-state fan facilitates the compact design of the cooling system inside the bore 4.

As shown in Fig. 2, the RF shielding mesh parts 121, 122 covers the air-in and air-out ducts provided in the housing. The size of each mesh part integrated with the RF shield may be designed to avoid RF signal, especially RF signal near Larmor frequency from leaking out, while remaining transparent to EM signals within the gradient (audio) frequency range. In one embodiment, the size of each mesh part may be kept below 4 cm × 4 cm, while being capacitively connected to other patches to cover the full volume of the fan.

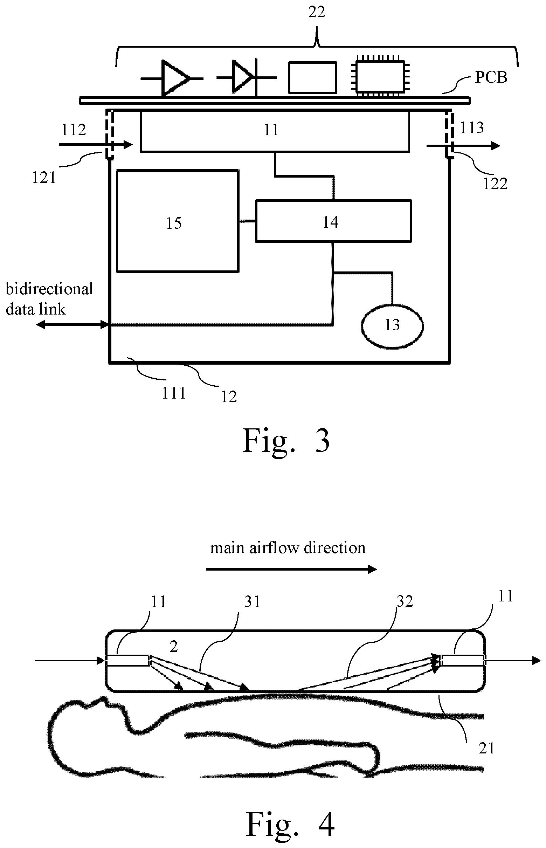

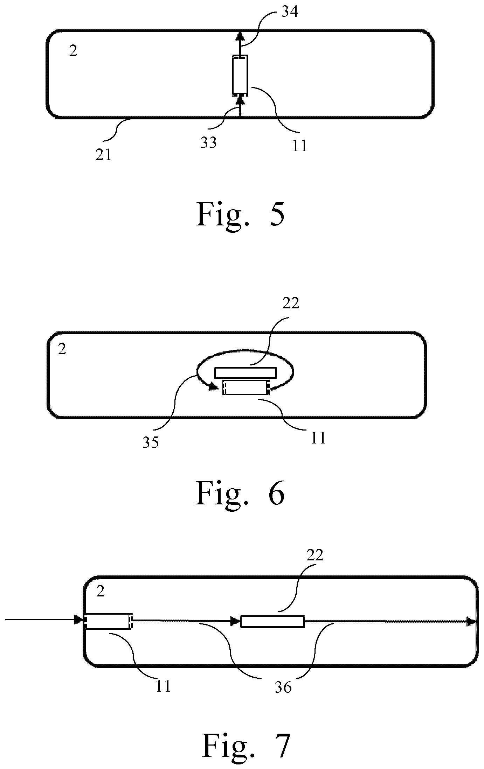

Fig. 3 depicts the block diagram of the cooling system according to another embodiment of the present invention. As shown in Fig. 3, the cooling system may further comprise a temperature measuring unit 13, a control unit 14, a power supply unit 15 and a RF filter unit (which is not shown in Fig. 5, at the position where the bidirectional link enters). These components are also provided inside the housing to enable local powering and control of the compact fan. The temperature measuring unit 13 may be configured to measure the local temperatures at a component to be cooled, the air-in duct, the air-out duct, and/or the ambient temperature within the bore. The temperature measuring unit 13 is connected to the control unit 14 so that the control unit 14 can regulate the operation of the compact fan 11 based on the temperature feedback.

In addition, the control unit may be configured to communicate with the MR apparatus via a bidirectional data link. On one hand, the control unit 14 may obtain information regarding the SAR, remote temperature model, and/or MR sequence from the MR apparatus for the operation of the compact fan. On the other hand, the control unit 14 may send interrupt information to the MR apparatus upon detecting irregular local temperatures to ensure safety. In some examples, the control unit 14 may connect to the electronics 22 of the in-bore equipment 2. For instance, when the in-bore equipment 2 is a coil array, the control unit 14 may raise a detuning error in the electronics 22 to interrupt the scan.

The power unit 15 may be configured to supply power to the compact fan 11, the temperature measuring unit 13, the controller unit 14 and the whole circuit of the cooling system. The RF filter unit 16 may be configured to further filter the RF signals to avoid electromagnetic interference.

As shown in Fig. 3, the compact fan 11 may be attached to a PCB which is part of the electronics 22 of the in-bore equipment. In this way, the air flows generated by the compact fan passes over the electronics 22 packaged on the PCB to achieve heat dissipation. Alternatively, the solid state fan is directly attached to the most dissipating part like a conventional heat sink. This embodiment allows for compact design of the cooling system by integrating it to the electronics.

Additionally, the compact fan 11 may be arranged at different positions of the in-bore equipment 2 and utilizes air channels 31-36 to enable localized cooling and ventilation. Figs 4 to 7 depict in-bore equipment with different arrangements of the compact fan according to examples of the present invention.

As shown in the left part of Fig. 4, when the in-bore equipment covers the body of the patient, the compact fan can be arranged in the in-bore equipment 2 with its air-in duct 112 adjacent to the outer periphery of the in-bore equipment 2. The air-out duct 113 and a surface 21 of the in-bore equipment 2 are connected by an air channel 31 to guide an air flow from the compact fan 11 to the surface 21.

In this embodiment, the compact fan may be positioned close to a side of the outer periphery, which faces the main airflow direction and the air-in duct of the compact fan may be arranged in parallel to this side so that the fresh air brought by the main airflow can reach the gap between the in-bore equipment and the patients. As shown in Fig. 4, multiple air channels 31 may be provided to communicate the air-out duct and different openings on the surface 21 to further enhance the cooling and ventilation effect.

The right part of Fig. 4 also depicts when the cooling system includes another compact fan 11 arranged in the in-bore equipment 2 with its air-out duct 113 adjacent to the outer periphery of the in-bore equipment 2. The air-in duct 112 of the other compact fan and the surface 21 of the in-bore equipment 2 are connected by an air channel 32 to guide an air flow from the surface 21 to the compact fan 11, In this example, warm air from the gap flows from the surface 21 to exit the bore 4 via the air channel 32 and the compact fan 11. As shown in Fig. 4, multiple air channels 32 may also be provided to communicate the air-in duct and different openings on the surface to further enhance the cooling and ventilation effect.

These two arrangements can be employed in combination or separately according to practical needs. Moreover, the in-bore equipment 2 can also be the patient bed 6 placed under that patient. If so, the surface 21 will be the upper horizontal surface of the patient bed 6 where the patient lays.

Fig. 5 shows another example when the compact fan 11 is arranged inside the in-bore equipment 2. In this example, the cooling system may comprise an air channel 33 connecting the air-in duct 112 and the surface 21 of the in-bore equipment, and another air channel 34 connecting the air-out duct 113 and an opposite surface of the in-bore equipment 2. The air flows from the surface 21 to the opposite side of the in-bore equipment via the air channels 33, 34 and the compact fan 11. Although not shown in Fig. 5, multiple air channels connecting the air-in/out ducts to the different positions on the surfaces can also be provided to enhance the cooling and ventilation effect.

In Fig. 5, the two surfaces are the opposite horizontal surfaces of the in-bore equipment 2. These two surfaces can also be adjacent surfaces. For instance, the air-in duct 112 may be connected to the bottom surface 21 of the in-bore equipment 2 while the air-out duct 113 is connected to the side surface close to the exit. Moreover, the compact fan 11 may also be arranged close to the electronics 22 inside the in-bore equipment 2 to achieve simultaneous heat dissipation. Different configurations can be employed according to practical needs.

Fig. 6 shows the example when the compact fan 11 is attached to the electronics 22 inside the in-bore equipment 2. The cooling system 1 may comprise an air channel 35 which connects the air-in duct 112 and air-out duct 113 of the compact fan and circulates the electronics 23 to guide an air flow around the electronics 22, in order to spread heat from local hotspots using the full volume/surface of the in-bore equipment for cooling. In some examples, airflow circulation can be created without any air channels. For instance, when a solid-state fan is used, it may be possible to adjust the airflow direction by modifying the electrode or piezoelectric configuration or by implementing programmable or active control systems.

Fig. 7 depicts an example of the present invention when the compact fan is arranged in the in-bore equipment 2 with its air-in duct 112 adjacent to the outer periphery of the in-bore equipment 2. The cooling system may comprise an air channel 36 connecting the air-out duct 113, electronics 22 of the in-bore equipment 2 and an opposite side of the in-bore equipment 2. The air flows from one side to the opposite side via the compact fan 11 and the air channel 36 to cool the electronics 22. In Fig. 7, these two sides are opposite sides along a radial direction of the bore 4. The side where in the air-in duct 112 locates may be the side away from the exit of the bore, while the opposite side may be the side close to the exit of the bore. However, the two sides can also be the opposite horizontal surfaces of the in-bore equipment. Different configurations can be employed according to practical needs.

The arrangements depicted in Fig. 4 to 7 may be used in combination or separately according to practical needs. When used together, the cooling system may comprise multiple compact fans arranged at different locations of the in-bore equipment 2. And the in-bore equipment 2 as depicted above may be used with or integrated into a medical imaging apparatus. For the MR apparatus comprising an in-bore equipment, the in-bore equipment can be at least one of the following: a coil array, a patient table, and/or an equipment for patient support. When applying the cooling system to a CT apparatus, the in-bore equipment may include for example a CT gantry, a patient table.

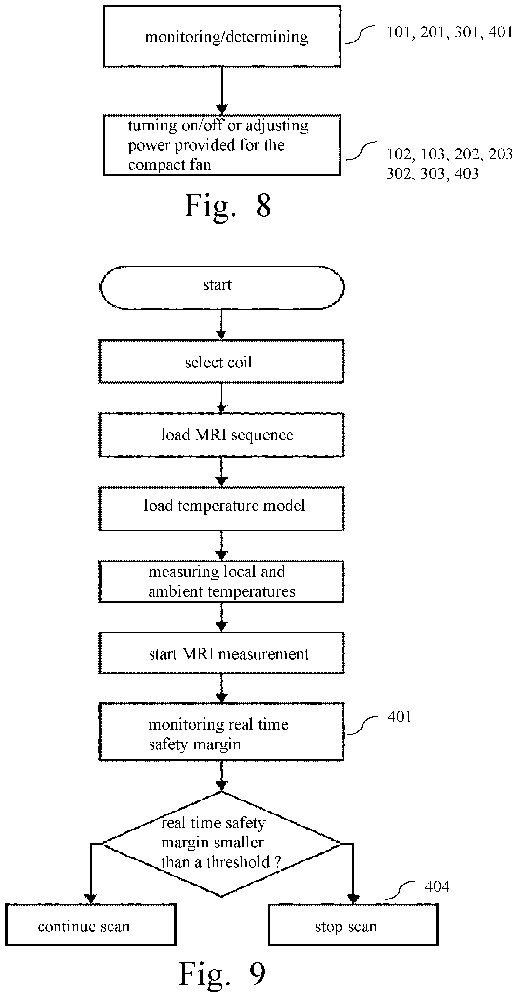

Fig. 8 depicts the method for operating the cooling system 1 of the MR apparatus discussed above according to different embodiments of the present invention. The methods can be computer-implemented and applied to the control unit 14 of the cooling system 1 or to a processor of the MR apparatus.

The cooling system may be controlled based on the local and ambient temperatures of the MR apparatus. In this embodiment, the method comprises:
101: monitoring local and/or ambient temperature of the MR apparatus,
102: switching the compact fan on or off based on the monitored temperature, and/or
103: adjusting the power provided for operating the compact fan based on the monitored temperature.

The local and ambient temperatures may be obtained from an external sensor or measured by the temperature measuring unit 13 of the compact fan. The local temperature may refer to the temperature of a component to be cooled, the temperature measured at the air-in and/or air-out duct of the compact fan, or similar locations. When the temperature is high, the fan may automatically be turned on, or the power of the fan may be increased to enhance cooling and ventilation. Conversely, when the temperature is low, the fan may be turned off, or the power of the fan may be decreased to conserve energy.

When the cooling system 1 comprises multiple compact fans 11, each compact fan may be controlled based on its local temperature.

According to another example, the cooling system 1 of the MR apparatus may be controlled based on a SAR of a scan session. the method may comprise the steps of:
201: monitoring a SAR of a scan, and
202: switching the compact fan on or off based on the monitored SAR, and/or
203: adjusting the power provided for operating the compact fan based on the monitored SAR.

For scans with higher SAR levels, the power of the compact fan may be increased to enhance cooling and ventilation. The SAR level may be obtained by the control unit via the bidirectional data link.

Optionally, the cooling system 1 may be controlled based on an MR sequence applied for the scan. In this embodiment, the method may comprise the steps of:
301: determining 301 an applied MR sequence, and
302: switching the compact fan on or off based on the applied MR sequence, and/or
303: adjusting the power provided for operating the compact fan according to the applied MR sequence.

Optionally, the cooling system 1 and the MR apparatus may also be controlled according to a remote temperature model. The method may comprise the steps of:
401: monitoring a real-time safety margin with respect to a remote temperature model, and
403: based on the real-time safety margin, adjusting the power of the compact fan by the control unit

The remote temperature model may refer to a system or method used to estimate or monitor the temperature of the MR apparatus without direct contact with the area being measured. In this example, the control unit 14 may calculate and monitor the real-time safety margin based on the difference between the real-time temperature and the maximum allowable temperature, adjusting the power of the compact fan accordingly. When the difference is large, the power provided for operating the compact fan may be decreased. Conversely, when the difference is small, the power provided for operating the compact fan may be increased to maintain the allowable safety margin. It ensures safe operation and avoids risks such as overheating, equipment damage, or harm to individuals.

In addition, when the real-time safety margin is smaller than a predetermined threshold, the methods may comprise the step of:
404: in response to detecting that the real-time safety margin is smaller than a threshold, sending information to the medical imaging device for interrupting operation.

The information to interrupt operation may be sent to the MR apparatus via the bidirectional data link. Moreover, the control unit 14 may directly interrupt the scan, for instance, by raising the detuning error of a coil array. The threshold may be set to be 1-5°C, which indicates that the temperature stages at least 1-5°C below the maximum allowable temperature.

For the sake of completeness, Fig. 9 depicts a complete procedure for operating the MR apparatus with the cooling system 1. In this procedure, the MR scanner selects coil, loads MRI sequence and temperature model. The control unit 14 of the cooling system may obtain the corresponding information and start monitoring local and ambient temperatures. After the MRI measurement starts, the control unit 14 may keep calculating and monitoring the real-time safety margin with respect to the temperature model. Once it is determined that the real-time safety margin is smaller than a threshold, the control units 14 may interrupt the scan by sending interruption to the MR scanner or by increasing the detuning error. Otherwise, the control units 14 may regulate the operation of the compact fan based on the local and ambient temperatures, the SAR and/or the MRI sequence during the scan.

It is understood that one or more of the aforementioned embodiments of the invention may be combined as long as the combined embodiments are not mutually exclusive.

In the context of the invention air-in duct and air-out duct, are equivalent to air inlet aperture and air outlet aperture, respectively.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to our advantage. Any reference signs in the claims should not be construed as limiting the scope. Further, for the sake of clearness, not all elements in the drawings may have been supplied with reference signs.

## Claims

1. A cooling system (1) for a medical apparatus, comprising:
a compact fan (11) which is resilient to magnetic interference,
a radio frequency, RF, shield (12) comprising an RF shielding housing (111) which completely encloses the compact fan (11), wherein
the housing (111) comprises an air-in duct (112) and an air-out duct (113), and RF shielding mesh parts (121, 122) covering the air-in duct (112) and the air-out duct (113).

2. The cooling system according to claim 1, wherein the compact fan (11) is a solid-state fan.

3. The cooling system according claim 1 or 2, further comprising:
a temperature measuring unit (13) for measuring local and/or ambient temperature in the medical apparatus,
a control unit (14) for controlling the compact fan (11),
a power supply unit (15) for supplying power to the compact fan (11), and/or
an RF filter unit (16), wherein
the control unit, the power supply unit and/or the RF filter unit (14, 15, 16) are arranged inside the housing (111).

4. An in-bore equipment (2) for a medical apparatus with a cooling system (1) according to any of claims 1 to 3.

5. The in-bore equipment (2) according to claim 4, wherein
the compact fan is arranged in the in-bore equipment (2) with its air-in duct (112) adjacent to the outer periphery of the in-bore equipment (2),
its air-out duct (113) and a surface (21) of the in-bore equipment (2) are connected by an air channel (31) to guide an air flow from the compact fan (11) to the surface (21); and
an additional or an alternative compact fan is arranged in the in-bore equipment (2) with its air-out duct (113) adjacent to the outer periphery of the in-bore equipment (2),
the air-in duct (112) of the additional or alternative compact fan and the surface (21) of the in-bore equipment (2) are connected by an air channel (32) to guide an air flow from the surface (21) to the compact fan (11),
wherein the surface (21) is adapted for forming an interface between the in-bore equipment (2) and a body portion of a patient who is to be examined.

6. The in-bore equipment according to claims 4, wherein
the compact fan is arranged in the interior of the in-bore equipment,
the air-in duct (112) of the compact fan and the surface (21) of the in-bore equipment are connected by an air channel (33), and
the air-out duct (113) of the compact fan and an opposite surface (22) are connected by another air channel (34) to guide an air flow from the surface (21) to the opposite surface (22).

7. The in-bore equipment according to claim 4, wherein
the in-bore equipment comprises electronics (23),
the compact fan is arranged in the interior of the in-bore equipment,
the cooling system comprises an air channel (35) which connects the air-in duct (112) and the air-out duct (113) of the compact fan and surrounds the electronics (23) to guide air flow around the electronics (22); and
an additional or an alternative compact fan is arranged in the in-bore equipment (2) with its air-in duct (112) adjacent to the outer periphery of the in-bore equipment (2),
the cooling system comprises an air channel (36) connecting the air-out duct (113) of the additional or alternative compact fan with the electronics (22) and an opposite side of the in-bore equipment (2) to guide an air flow passing the electronics (22).

8. The in-bore equipment according to any of claims 4 to 7, comprising multiple compact fans (11) arranged at different locations of the in-bore equipment (2).

9. A medical apparatus comprising an in-bore equipment (2) according to any one of clams 4 to 8.

10. The medical apparatus according to claim 9, wherein the medical apparatus is a magnetic resonance, MR, apparatus.

11. The medical apparatus according to claim 9, wherein the medical apparatus is one of a computed tomography apparatus, a single photon emission computed tomography apparatus and a positron emission tomography apparatus.

12. The medical apparatus of any of claims 9 to 11, wherein the in-bore equipment (2) comprises at least one of the following: a coil array, a patient table, and an equipment for patient support.

13. A method of operating a medical apparatus according to any of claims 9 to 12, comprising:
based on monitoring (101) local and/or ambient temperature of the medical apparatus, switching (102) the compact fan on or off, and/or adjusting (103) the power provided for operating the compact fan; and/or
based on monitoring (201) a specific absorption rate, SAR, of a scan, switching (202) the compact fan on or off, and/or adjusting (203) the power provided for operating the compact fan; and/or
based on determining (301) an applied MR sequence, switching (302) the compact fan on or off, and/or adjusting (303) the power provided for operating the compact fan according to the applied MR sequence.

14. The method according to claim 13, further comprising:
monitoring (401) a real-time safety margin with respect to a remote temperature model, and
adjusting (403) the power provided for operating the compact fan, based on the real-time safety margin.

15. The method according to claim 14, further comprising:
in response to detecting that the real-time safety margin is smaller than a threshold, sending (404) information to the medical apparatus to interrupt operation.
